# EUROPÄISCHE PATENTSCHRIFT

(11) **EP 3 448 259 B1**
(45) Veröffentlichungstag und Bekanntmachung des Hinweises auf die Patenterteilung: **12.08.2020**
(21) Anmeldenummer: 17723282.4
(22) Anmeldetag: 25.04.2017
(51) Int. Cl.: A61B 5/15, A61M 39/02, A61M 39/24

(54) **ENTNAHMEPORT ZUM ENTNEHMEN EINER FLÜSSIGKEIT, SYSTEM UND VERWENDUNG EINES ENTNAHMEPORTS**
REMOVAL PORT FOR REMOVING A FLUID, SYSTEM AND USE OF A REMOVAL PORT
ORIFICE DE PRÉLÈVEMENT DESTINÉ AU PRÉLÈVEMENT D'UN LIQUIDE, SYSTÈME ET UTILISATION D'UN PORT DE PRÉLÈVEMENT

(30) Priorität: 27.04.2016 DE 102016005072
(43) Veröffentlichungstag der Anmeldung: 06.03.2019
(73) Patentinhaber: Haindl, Hans, 30974 Wennigsen (DE)
(72) Erfinder: Haindl, Hans, 30974 Wennigsen (DE)
(74) Vertreter: Vossius & Partner Patentanwälte Rechtsanwälte mbB
(86) Internationale Anmeldenummer: PCT/EP2017/059755
(87) Internationale Veröffentlichungsnummer: WO 2017/186695

(56) Entgegenhaltungen:
- EP-A1- 0 240 590
- WO-A1-00/40291
- WO-A1-2008/101025
- WO-A1-2015/117126
- JP-A- 2012 005 605

## Beschreibung

Die Erfindung betrifft einen Entnahmeport zum Entnehmen einer Flüssigkeit, insbesondere Blut, aus einer Schlauchleitung.

Bei verschiedenen medizinischen Behandlungen, beispielsweise bei der Herzkatheterisierung oder in der Intensivmedizin, kann es erforderlich sein, einem Patienten häufig Blut zu entnehmen. Dies kann beispielsweise erforderlich sein, um das entnommene Blut zu analysieren. Bei derartigen Behandlungen hat der Patient häufig eine arterielle oder venöse Kanüle in einem Blutgefäß liegen. Um den Patienten nicht für jede Blutentnahme neu punktieren zu müssen, ist es wünschenswert, zu Analysezwecken das Blut aus der Schlauchleitung zu entnehmen.

Im Stand der Technik ist zu diesem Zweck beispielsweise ein Dreiwegehahn bekannt, mit dem eine Infusionsleitung zu einer Infusionsflasche hin abgesperrt wird und dann zunächst Infusionslösung und später Blut aus dem Schenkel des Dreiwegehahnes entnommen werden kann. Ein Problem bei herkömmlichen Dreiwegehähnen besteht dabei darin, dass zunächst das mit Infusionslösung verdünnte Blut entnommen werden muss, um eine Verfälschung von Laborergebnissen zu vermeiden. Deshalb muss zunächst mit einer Spritze eine Portion dieses Mischblutes abgezogen werden. Sobald unverdünntes Blut vermutet wird, wird die Spritze gewechselt und dann das unverdünnte Blut abgezogen und zur Analyse verwendet. Das abgezogene Mischblut wird verworfen. Dies führt dazu, dass dem Patienten jedes Mal mehr Blut entnommen werden muss, als für die Analyse eigentlich nötig ist. Dies kann unter Umständen im Laufe der Behandlung zu Blutarmut führen. Weiterhin sind derartige Techniken auch hygienisch bedenklich, da in dem zur Blutabnahme benutzten Schenkel des Dreiwegehahns Blutreste stehen bleiben können, in denen sich Keime vermehren können.

Um diese erhöhten Blutverluste zu reduzieren oder vollständig zu unterbinden, können verschiedene Systeme eingesetzt werden, bei denen das abgezogene Mischblut in einem geschlossenen System an den Patienten zurückgegeben wird. Zu diesen Systemen gehört beispielsweise ein System, wie es in der EP 1 017 317 A1 beschrieben ist.

Derartige Systeme bestehen aus einem Flüssigkeitsreservoir, das in einer Leitung zur Gefäßkanüle angeordnet ist. Durch dieses Flüssigkeitsreservoir läuft die Infusionslösung zum Patienten. In dieses Reservoir lässt sich, beispielsweise durch Ziehen eines Kolbens in einem Zylinder, Flüssigkeit aus der Schlauchleitung heraus ziehen. Es wird dabei so viel Flüssigkeit aus der Schlauchleitung herausgezogen, bis der Schenkel der Schlauchleitung vom Reservoir zum Patienten vollständig mit unverdünntem Blut gefüllt ist. Auf diesem Schlauchschenkel befindet sich ein Entnahmeport, in den beispielsweise mit einer speziellen Kanüle oder direkt mit dem Spritzenkegel eingestochen und Blut entnommen werden kann, wobei sich der Port nach der Blutentnahme automatisch wieder verschließt. Danach wird das Reservoir, beispielsweise durch ein Vorschieben des Kolbens, wieder entleert und das Mischblut an den Patienten zurückgegeben. Ein Vorteil dieser Anordnung ist, dass der Patient das nicht benötigte Blut wieder bekommt und dass typischerweise nicht signifikante Blutreste in einem Totraum, wie etwa bei einem Dreiwegehahn, stehen bleiben und dadurch eine Infektionsgefahr besteht. Die Entnahmeports zur Blutentnahme lassen sich meist problemlos desinfizieren.

Wenn ein solcher Entnahmeport zur Blutentnahme in der Schlauchleitung angeordnet ist, muss sichergestellt sein, dass dieser das Blut tatsächlich patientenseitig anzieht, also unverdünntes Blut, und dass nicht etwa Blut von der patientenabgewandten Seite, also Mischblut, aus dem Reservoir angezogen wird. Zu diesem Zweck wird häufig an der patientenabgewandten Seite des Entnahmeports ein Absperrhahn angeordnet. Bei manchen Entnahmeports ist ein derartiger Absperrhahn auch in das Gehäuse des Entnahmeports integriert. Ein solcher Entnahmeport wird beispielsweise in der EP 1 234 596 A1 beschrieben.

Derartige Absperrhähne müssen von medizinischem Personal bedient werden. Dennoch besteht das Risiko einer fehlerhaften Bedienung. Beispielsweise kann es vorkommen, dass vergessen wird, den Absperrhahn zu schließen oder nach der Blutentnahme anschließend wieder zu öffnen. Im ersteren Fall kann das Blut von der patientenfernen Seite angesogen werden, was zu fehlerhaften Analysenergebnissen führen kann. Entnahmeports mit integriertem Hahn weisen ein geringeres Risiko einer fehlerhaften Bedienung auf, weil ohne Umschalten des Hahnes keine Entnahme möglich ist, sind aber immer noch mit Nachteilen verbunden. Beispielsweise weisen Spritzenkegel erhebliche Toleranzen in Länge und Durchmesser auf. Da der Hahn als starres Bauelement aber relativ nahe unter einer Membran des Ports angeordnet ist, kann es vorkommen, dass bestimmte Spritzenkegel auf den Hahn aufstoßen und deshalb in der Führung des Entnahmeports nicht ordnungsgemäß klemmen, so dass es zu unbeabsichtigter Ablösung der Spritze vom Entnahmeport (so genannte "Diskonnektion") während der Blutabnahme kommen kann.

Die EP 0 240 590 A1 offenbart ein Injektions-Absperrventil.

Die JP 2012 005 605 A offenbart ein Verbindungselement für eine Strömungspassage eines Wirkstoffs.

Die WO 2008/101025 A1 offenbart ein System zum Entnehmen von Blutproben.

Die WO 2015/117126 A1 offenbart ein medizinisches Ventil.

Die WO 00/40291 A1 offenbart ein Ventil zur Injektion oder Probenentnahme von Blut.

Es ist daher eine Aufgabe der vorliegenden Erfindung, einen verbesserten Entnahmeport zur Blutentnahme bereitzustellen, der den oben beschriebenen Problemen Rechnung trägt.

Diese und weitere Aufgaben werden mit einem Entnahmeport, einem System und einer Verwendung gemäß den unabhängigen Ansprüchen gelöst. Weitere bevorzugte Merkmale sind in den abhängigen Ansprüchen beschrieben.

Ein erfindungsgemäßer Entnahmeport ist zum Entnehmen einer Flüssigkeit, insbesondere von Blut, aus einer Schlauchleitung geeignet. Der Entnahmeport weist einen ersten Schlauchanschluss und einen zweiten Schlauchanschluss auf. Der erste Schlauchanschluss kann mit einem patientenseitigen Schenkel einer Schlauchleitung verbunden sein oder verbindbar sein. Der zweite Schlauchanschluss kann beispielsweise mit einen zu einem Flüssigkeitsreservoir führenden patientenabgewandten Schenkel verbunden oder verbindbar sein, wobei auch andere Verwendungen des erfindungsgemäßen Entnahmeports möglich sind.

Der Entnahmeport weist eine Fluidverbindung, insbesondere eine Flüssigkeitsverbindung, zwischen dem ersten Schlauchanschluss und dem zweiten Schlauchanschluss auf. Der Entnahmeport kann einen Aufnahmeabschnitt aufweisen. Der Aufnahmeabschnitt kann dazu geeignet sein, einen Spritzenabschnitt einer Spritze reversibel lösbar aufzunehmen. Der Spritzenabschnitt kann insbesondere ein Spritzenkegel sein. Der Entnahmeport ist derart ausgestaltet, dass ein Einführen des Spritzenabschnitts in den Aufnahmeabschnitt die Flüssigkeitsverbindung zwischen dem ersten Schlauchanschluss und dem zweiten Schlauchanschluss unterbricht. Der Entnahmeport kann derart ausgestaltet sein, dass das Einführen des Spritzenabschnitts in den Aufnahmeabschnitt eine fluidische Kommunikation, insbesondere eine Flüssigkeitskommunikation, des Spritzenabschnitts, beispielsweise des Spritzenkegels, mit dem ersten Schlauchanschluss bewirkt.

Bei einem derartigen Entnahmeport bewirkt das Einführen des Spritzenkegels in den Aufnahmeabschnitt des Entnahmeports automatisch, dass die Fluidverbindung zwischen dem ersten Schlauchanschluss und dem zweiten Schlauchanschluss unterbrochen wird. Die Fluidverbindung kann insbesondere eine Flüssigkeitsverbindung sein. Durch das Unterbrechen der Flüssigkeitsverbindung wird eine Strömung von Flüssigkeit zwischen dem ersten Schlauchanschluss und dem zweiten Schlauchanschluss im Wesentlichen unterbunden. Das Risiko, dass unbeabsichtigt Mischblut aus einem Reservoir mit Mischblut angesogen wird, wird verringert. Im Idealfall besteht kein Risiko mehr, dass unbeabsichtigt Flüssigkeit aus einem anderen Schenkel als dem ersten Schenkel der Schlauchleitung angesogen wird. Aufgrund des verminderten Risikos von Handhabungsfehlem wird das Risiko einer Verfälschung von Analyseergebnissen bei einer weiteren Analyse des Bluts verringert.

Die Unterbrechung der Fluidverbindung zwischen dem ersten Schlauchanschluss und dem zweiten Schlauchanschluss erfordert nicht zwingend, dass der zweite Schlauchanschluss vollständig fluidisch von dem ersten Schlauchanschluss getrennt ist. Es genügt, dass ein Strömungswiderstand für einen Flüssigkeitsstrom zwischen dem ersten und zweiten Schlauchanschluss größer, vorteilhaft viel größer, ist als ein Strömungswiderstand zwischen dem ersten Schlauchanschluss und dem Spritzenkegel, wenn der Spritzenkegel in den Entnahmeport eingeführt ist. Es ist jedoch bevorzugt, dass der Flüssigkeitsstrom (d.h. insbesondere der Blutstrom) zwischen dem ersten und zweiten Schlauchanschluss substantiell und besonders bevorzugt vollständig unterbrochen bzw. unterbunden wird.

Der Entnahmeport weist bevorzugt ein Gehäuse und einen Verschlussabschnitt auf. Der Verschlussabschnitt kann eingerichtet sein, um mit dem Spritzenabschnitt reversibel lösbar in Kontakt zu treten, um die Fluidverbindung zwischen dem ersten Schlauchanschluss und dem zweiten Schlauchanschluss zu unterbrechen. Die Unterbrechung der Fluidverbindung kann dabei beispielsweise dadurch erfolgen, dass der Verschlussabschnitt unter Einwirkung des Spritzenkegels verformt wird und/oder dass der Spritzenkegel und der Verschlussabschnitt anderweitig so zusammenwirken, dass die Fluidverbindung zwischen dem ersten und dem zweiten Schlauchanschluss unterbrochen wird, während der Spritzenkegel in einer Flüssigkeitsverbindung mit dem ersten Schlauchanschluss steht.

Der Verschlussabschnitt kann eine Dichtfläche an dem Gehäuse des Entnahmeports aufweisen. Die Dichtfläche kann so angeordnet sein, dass bei einem Kontakt des Spritzenabschnitts mit der Dichtfläche der zweite Schlauchanschluss verschlossen wird, beispielsweise im Wesentlichen flüssigkeitsdicht verschlossen wird. Der dichtende Eingriff zwischen dem Spritzenabschnitt und dem Gehäuse kann beispielsweise sichergestellt werden, indem wenigstens ein Teil einer Innenwand des Gehäuses sich so stark verjüngt, dass der eingeführte Spritzenkegel sicher entlang einer durchgängigen, beispielsweise im Wesentlichen ringförmigen Kontaktlinie die Innenwand berührt.

Der Entnahmeport kann so ausgestaltet sein, dass der erste Schlauchanschluss eine erste Längsachse und der zweite Schlauchanschluss eine zu der ersten Längsachse versetzte zweite Längsachse aufweist. Eine derartige Anordnung des ersten und zweiten Schlauchanschlusses erlaubt es, in einem Entnahmeport mit einer einfachen Geometrie die Fluidverbindung zwischen dem ersten Schlauchanschluss und dem zweiten Schlauchanschluss durch den Kontakt des Spritzenkegels mit der Dichtfläche zu unterbrechen. Die erste Längsachse des ersten Schlauchanschlusses und die zweite Längsachse des zweiten Schlauchanschlusses können zueinander parallel verlaufen. Die erste Längsachse des ersten Schlauchanschlusses und die zweite Längsachse des zweiten Schlauchanschlusses können vorteilhaft zueinander entlang einer Einführrichtung versetzt sein, entlang der der Spritzenkegel in den Entnahmeport einführbar ist.

Das Gehäuse kann ausgestaltet sein, einen Flüssigkeitsstrom zwischen dem ersten Schlauchanschluss und dem zweiten Schlauchanschluss umzulenken, wenn der Spritzenabschnitt nicht in den Aufnahmeabschnitt eingeführt ist. Das Gehäuse kann dazu wenigstens eine quer, insbesondere im Wesentlichen senkrecht, zu der ersten Längsachse des ersten Schlauchanschlusses verlaufende Wand aufweisen.

Der Verschlussabschnitt kann bevorzugt elastisch verformbar sein, um bei dem Einführen des Spritzenabschnitts in den Aufnahmeabschnitt den zweiten Schlauchanschluss zu verschließen, insbesondere im Wesentlichen flüssigkeitsdicht zu verschließen. Der Verschlussabschnitt kann einen eine Längsachse des zweiten Schlauchanschlusses umlaufenden, beispielsweise im Wesentlichen kegelstumpfförmigen, Bereich aufweisen, der beim Einführen des Spritzenabschnitts von dem Spritzenabschnitt im Wesentlichen abdichtend zusammengedrückt wird.

Um sicherzustellen, dass der Spritzenabschnitt auch bei Kontakt mit dem elastisch verformbaren Verschlussabschnitt noch in guter fluidischer Kommunikation mit dem ersten Schlauchanschluss steht, kann das Gehäuse eine Einführrichtung für den Spritzenkegel derart festlegen, dass die Einführrichtung sowohl zu einer ersten Längsachse des ersten Schlauchanschlusses als auch zu einer zweiten Längsachse des zweiten Schlauchanschlusses geneigt ist.

Das Gehäuse kann bevorzugt die Einführrichtung für den Spritzenkegel derart festlegen, dass die Einführrichtung mit der ersten Längsachse des ersten Schlauchanschlusses einen stumpfen Winkel einschließt. Das Gehäuse kann die Einführrichtung für den Spritzenkegel derart festlegen, dass die Einführrichtung mit der zweiten Längsachse des zweiten Schlauchanschlusses einen spitzen Winkel einschließt. Der Winkel kann größer als 60°, beispielsweise von 60 bis 90° sein.

Der elastisch verformbare Verschlussabschnitt kann durch das Einführen des Spritzenabschnitts in den Aufnahmeabschnitt reversibel aus einer ersten Konfiguration in eine zweite Konfiguration überführt werden. In der ersten Konfiguration kann der Verschlussabschnitt so in dem Gehäuse des Entnahmeports angeordnet sein, dass der Verschlussabschnitt sowohl den ersten Schlauchanschluss als auch den zweiten Schlauchanschluss frei lässt, um eine Flüssigkeitsverbindung dazwischen zu ermöglichen. In der zweiten Konfiguration kann der elastisch verformbare Verschlussabschnitt den zweiten Schlauchanschluss verschließen, während vorteilhaft eine fluidische Kommunikation zwischen dem ersten Schlauchanschluss und dem Spritzenkegel hergestellt wird.

Das Gehäuse kann eine Öffnung aufweisen, über die der Spritzenabschnitt in den Aufnahmeabschnitt des Entnahmeports einführbar ist. Der elastisch verformbare Verschlussabschnitt kann eingerichtet sein, um in der ersten Konfiguration die Öffnung zu verschließen und/oder abzudichten. Dadurch kann in einfacher Weise das Risiko einer Kontamination des Entnahmeports auch ohne zusätzliche Komponenten verhindert werden.

Der elastisch verformbare Verschlussabschnitt kann aus einem elastomeren Material bestehen oder kann ein elastomeres Material aufweisen. Das elastomere Material kann beispielsweise Silikon, Latex, ein thermoplastisches Elastomer (TPE) und/oder Ethylen-Propylen-DienKautschuk (EPDM) aufweisen.

Der Verschlussabschnitt kann eingerichtet sein, um den in den Aufnahmeabschnitt eingeführten Spritzenabschnitt allein oder im Zusammenspiel mit dem Gehäuse, insbesondere im Zusammenspiel mit einem weiblichen Kegelabschnitt, im Inneren des Gehäuses reibschlüssig zu halten. Alternativ kann der Spritzenabschnitt auch vom Gehäuse allein gehalten werden. Der Verschlussabschnitt kann eingerichtet sein, um den in den Aufnahmeabschnitt eingeführten Spritzenabschnitt klemmend zu halten. Das Risiko, dass sich die Spritze unbeabsichtigt vom Entnahmeport löst, wird dadurch verringert.

Das Gehäuse des Entnahmeports weist eine Öffnung auf, über die der Spritzenabschnitt in den Aufnahmeabschnitt des Entnahmeports einführbar ist. Der Entnahmeport weist eine an der Öffnung angeordnete Dichtung auf. Die Dichtung kann für einen dichtenden Eingriff mit Spritzenkegeln unterschiedlicher Durchmesser elastisch verformbar sein. Alternativ oder zusätzlich kann das Gehäuse an der Öffnung für einen dichtenden Eingriff mit Spritzenkegeln unterschiedlicher Durchmesser elastisch verformbar sein.

Der Entnahmeport ist eingerichtet, den Spritzenabschnitt reibschlüssig, insbesondere mit einer radial nach innen gerichteten klemmenden Kraft, am Entnahmeport zu halten, wenn der Spritzenabschnitt in den Aufnahmeabschnitt eingeführt ist.

Der Entnahmeport kann eine Halterung zum Halten der Dichtung aufweisen. Die Halterung kann als Deckel ausgebildet sein, der die Dichtung teilweise übergreift. Die Halterung kann eingerichtet sein, den Spritzenabschnitt reibschlüssig, insbesondere mit einer radial nach innen gerichteten klemmenden Kraft, an dem Entnahmeport zu halten, wenn der Spritzenabschnitt in den Aufnahmeabschnitt eingeführt ist. Alternativ oder zusätzlich kann die Dichtung eingerichtet sein, den Spritzenabschnitt reibschlüssig, insbesondere mit einer radial nach innen gerichteten klemmenden Kraft, an dem Entnahmeport zu halten, wenn der Spritzenabschnitt in den Aufnahmeabschnitt eingeführt ist.

Die Dichtung kann aus einem Elastomer, beispielsweise Silikon, Latex, TPE und/oder EPDM, bestehen.

Die Dichtung weist einen Vorsprung, beispielsweise eine Nase, für eine abdichtende Anlage an einer Oberfläche des Gehäuses auf. Die Nase kann so vorgesehen sein, dass sie sich von einer Innenseite des Gehäuses in die Öffnung erstreckt. Eine Außenoberfläche der Nase ist im Wesentlichen bündig mit einer Außenoberfläche des Gehäuses, wenn der Spritzenkegel nicht in den Aufnahmeabschnitt eingeführt ist.

Der in die Öffnung hineinragende Vorsprung der Dichtung bildet einen stufenlosen Übergang zwischen der Außenoberfläche der Dichtung und der umgebenden Außenoberfläche des Gehäuses und/oder der umgebenden Außenoberfläche der die Dichtung haltenden Halterung. Auf diese Weise wird in der Umgebung der Öffnung eine stufenlose Außenoberfläche des Entnahmeports für eine Wischdesinfektion bereitgestellt, wenn der Spritzenabschnitt nicht in den Entnahmeport eingeführt ist. Es entsteht eine stufenlose, gut desinfizierbare Außenoberfläche des Entnahmeports.

Das Gehäuse und/oder die Dichtung können eingerichtet sein, um den Spritzenabschnitt an einem von einem Ende des Spritzenkegels beabstandeten Abschnitt des Spritzenkegels reibschlüssig zu halten. Das Gehäuse und/oder die Dichtung können eingerichtet sein, um den Spritzenabschnitt an einem von einem Ende des Spritzenkegels beabstandeten Abschnitt des Spritzenkegels festzuklemmen und/oder klemmend zu halten. Das Risiko, dass sich die Spritze unbeabsichtigt vom Entnahmeport löst, wird dadurch verringert.

Der Entnahmeport kann eingerichtet sein, den Spritzenabschnitt klemmend zu halten, ohne dass der Spritzenabschnitt mit dem Entnahmeport verschraubt und/oder anderweitig formschlüssig gekoppelt wird.

Der Entnahmeport kann eingerichtet sein, um den Spritzenabschnitt wenigstens an einem Endbereich des Spritzenabschnitts innerhalb des Gehäuses reibschlüssig zu halten. Der Entnahmeport kann eingerichtet sein, um den Spritzenabschnitt wenigstens an einem Endbereich des Spritzenabschnitts innerhalb des Gehäuses festzuklemmen und/oder klemmend zu halten. Das Risiko, dass sich die Spritze unbeabsichtigt vom Entnahmeport löst, wird dadurch verringert.

Das Gehäuse kann Vorsprünge, insbesondere Rippen, zum Führen oder reibschlüssigen Halten des Spritzenabschnitts aufweisen. Die Vorsprünge können an der Öffnung, beispielsweise an wenigstens zwei Positionen umfangs um die Öffnung, vorgesehen sein, um den Spritzenkegel zu führen. Die Vorsprünge können eine Einführrichtung definieren, in der der Spritzenkegel in den Entnahmeport einführbar ist.

Der Entnahmeport kann wenigstens eine Halteplatte zum Halten und/oder Fixieren des Entnahmeports aufweisen. Die Halteplatte kann geeignet sein, um den Entnahmeport auf der Haut eines Patienten oder an einer anderen Struktur, beispielsweise einer Struktur eines Bettes, eines Operationstisches oder eines Infusionsständers zu fixieren. Die Halteplatte kann so bemessen sein, dass der Entnahmeport an der Halteplatte mit zwei Fingern gehalten werden kann, während der Spritzenkegel eingeführt wird.

Der Entnahmeport nach jedem der offenbarten Ausführungsbeispiele kann eingerichtet sein, um eine Blutentnahme ohne wesentliche Einschränkungen des Durchströmungsquerschnittes zu ermöglichen. Der Entnahmeport kann derart eingerichtet sein, dass der Durchströmungsquerschnitt zwischen dem ersten Schlauchanschluss und dem Aufnahmeabschnitt nicht verringert wird, wenn der Spritzenabschnitt in den Aufnahmeabschnitt eingeführt wird. Der Entnahmeport kann alternativ oder zusätzlich derart eingerichtet sein, dass der Entnahmeport die Spritzenöffnung nicht überdeckt, wenn der Spritzenabschnitt vollständig in den Aufnahmeabschnitt eingeführt ist. Insbesondere kann der Entnahmeport derart eingerichtet sein, dass die Dichtung des Entnahmeports von der Spritzenöffnung beabstandet an einer Außenoberfläche des Spritzenkegels anliegt, wenn der Spritzenabschnitt vollständig in den Aufnahmeabschnitt eingeführt ist.

Der Entnahmeport nach jedem der offenbarten Ausführungsbeispiele kann derart eingerichtet sein, dass sich der Entnahmeport nach Entfernen des Spritzenabschnitts aus dem Entnahmeport selbst wieder freispült. Der Entnahmeport kann hierzu eine Nase am patientenfernen Eingang aufweisen.

Ein erfindungsgemäßes System weist den erfindungsgemäßen Entnahmeport und eine Spritze auf. Die Spritze weist einen Spritzenkegel auf, der dazu geeignet ist, zum Entnehmen von Blut aus einer Schlauchleitung über den Entnahmeport reversibel lösbar in den Aufnahmeabschnitt des Entnahmeports eingeführt zu werden.

Bei einem derartigen System bewirkt das Einführen des Spritzenkegels in den Aufnahmeabschnitt des Entnahmeports automatisch, dass die Fluidverbindung zwischen dem ersten und zweiten Schlauchanschluss unterbrochen wird. Das Risiko, dass unbeabsichtigt Mischblut aus einem Reservoir mit Mischblut angesogen wird, wird dadurch verringert. Aufgrund des verminderten Risikos von Handhabungsfehlern wird ferner das Risiko einer Verfälschung von Analyseergebnissen bei einer weiteren Analyse des Bluts verringert.

Das System kann so ausgestaltet sein, dass das System durch das Einführen des Spritzenkegels in den Aufnahmeabschnitt zwischen einer ersten Konfiguration, in der der Spritzenkegel außerhalb des Aufnahmeabschnitts angeordnet ist und die Fluidverbindung zwischen dem ersten Schlauchanschluss und dem zweiten Schlauchanschluss offen ist, und einer zweiten Konfiguration überführbar ist. In der zweiten Konfiguration des Systems ist der Spritzenkegel bis zu einem Anschlag in den Aufnahmeabschnitt des Entnahmeports eingeführt, die Fluidverbindung zwischen dem ersten Schlauchanschluss und dem zweiten Schlauchanschluss ist unterbrochen, und der Spritzenkegel kommuniziert fluidisch mit dem ersten Schlauchanschluss.

Das System kann einen ersten Schenkel einer Schlauchleitung aufweisen, der mit dem ersten Schlauchanschluss verbunden ist. Das System kann einen zweiten Schenkel einer Schlauchleitung aufweisen, der mit dem zweiten Schlauchanschluss verbunden ist. Der zweite Schenkel der Schlauchleitung kann mit einem Flüssigkeitsreservoir verbunden sein, das beispielsweise eine Mischung aus Blut und einer Infusionslösung enthalten kann.

Bei einem erfindungsgemäßen Verfahren wird der erfindungsgemäße Entnahmeport zum Entnehmen von Blut aus einer Schlauchleitung verwendet.

Bei dem erfindungsgemäßen Entnahmeport, dem erfindungsgemäßen System und der erfindungsgemäßen Verwendung kann die Fluidverbindung zwischen dem ersten und zweiten Schlauchanschluss jeweils eine Flüssigkeitsverbindung sein. Diese kann unterbrochen werden, so dass im Wesentlichen kein Flüssigkeitsstrom mehr zwischen dem ersten und dem zweiten Schlauchanschluss mehr möglich ist, wenn der Spritzenkegel bis zu einem Anschlag in den Entnahmeport eingeführt ist. Die gleichzeitig ermöglichte fluidische Kommunikation zwischen dem Spritzenkegel und dem ersten Schlauchanschluss kann eine Flüssigkeitskommunikation sein, die es ermöglicht, Flüssigkeit über den ersten Schlauchanschluss in den Spritzenkegel einzusaugen.

Bevorzugte Ausführungsformen der erfindungsgemäßen Sicherheitskanüle werden nachfolgend mit Bezug auf die Figuren im Detail beschrieben. Es zeigen:
- Fig. 1: eine Schnittansicht eines erfindungsgemäßen Entnahmeports, wenn eine Fluidverbindung zwischen einem ersten und zweiten Schlauchanschluss offen ist;
- Fig. 2: eine weitere Schnittansicht des Entnahmeports gemäß Figur 1, wenn die Fluidverbindung zwischen dem ersten und zweiten Schlauchanschluss offen ist;
- Fig. 3: eine Schnittansicht des Entnahmeports gemäß Figur 1, wenn die Fluidverbindung zwischen dem ersten und zweiten Schlauchanschluss unterbrochen ist;
- Fig. 4: eine weitere Schnittansicht des Entnahmeports gemäß Figur 1, wenn die Fluidverbindung zwischen dem ersten und zweiten Schlauchanschluss unterbrochen ist;
- Fig. 5: eine Schnittansicht eines erfindungsgemäßen Entnahmeports, wenn eine Fluidverbindung zwischen einem ersten und zweiten Schlauchanschluss offen ist;
- Fig. 6: eine weitere Schnittansicht des Entnahmeports gemäß Figur 5 entlang einer in Figur 5 dargestellten Schnittlinie VI-VI, wenn die Fluidverbindung zwischen dem ersten und zweiten Schlauchanschluss offen ist;
- Fig. 7: eine Schnittansicht des Entnahmeports gemäß Figur 5, wenn die Fluidverbindung zwischen dem ersten und zweiten Schlauchanschluss unterbrochen ist;
- Fig. 8: eine weitere Schnittansicht des Entnahmeports gemäß Figur 5 entlang der in Figur 7 dargestellten Schnittlinie VIII-VIII, wenn die Fluidverbindung zwischen dem ersten und zweiten Schlauchanschluss unterbrochen ist;
- Fig. 9: eine Perspektivansicht einer Halterung einer Dichtung des Entnahmeports gemäß Figur 1;
- Fig. 10: eine weitere Perspektivansicht einer Halterung einer Dichtung des Entnahmeports gemäß Figur 1; und
- Fig. 11: eine teilweise Schnittansicht des Entnahmeports gemäß Figur 1.

Nachfolgend werden bevorzugte und vorteilhafte Ausführungsbeispiele der Erfindung unter Bezugnahme auf die Figuren beschrieben, in denen identische Bezugszeichen identische oder ähnliche Elemente bezeichnen. Die Merkmale der verschiedenen Ausführungsbeispiele können miteinander kombiniert werden, sofern dies in der nachfolgenden Beschreibung nicht ausdrücklich ausgeschlossen wird.

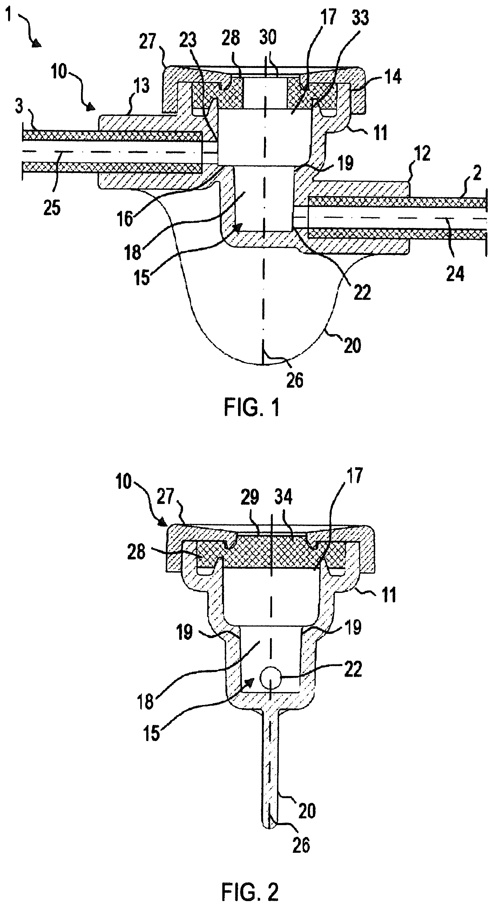

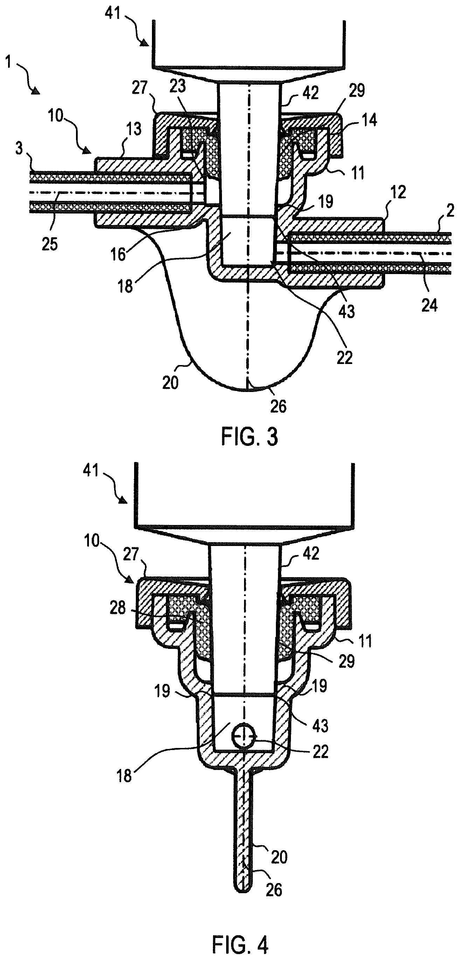

Die Figuren 1 bis 4 zeigen ein System 1 mit einem Entnahmeport 10 nach einer Ausführungsform. Der Entnahmeport 10 erlaubt ein Abziehen von Blut oder einer anderen Flüssigkeit aus einem ersten Schenkel 2 einer Schlauchleitung. Figur 1 zeigt den Entnahmeport 10 in einer Schnittansicht, wobei die Schnittebene parallel zu Längsachsen 24, 25 von Schlauchanschlüssen 12, 13 verläuft und das System 1 in einem Zustand dargestellt ist, in dem eine Fluidverbindung offen ist. Die Fluidverbindung kann insbesondere eine Flüssigkeitsverbindung 15 sein. Figur 2 zeigt den Entnahmeport in einer Schnittansicht, wobei die Schnittebene senkrecht zu den Längsachsen 24, 25 der Schlauchanschlüsse 12, 13 verläuft und das System 1 in einem Zustand dargestellt ist, in dem die Flüssigkeitsverbindung 15 offen ist. Figur 3 und Figur 4 zeigen den Entnahmeport in einer den Schnittansichten von Figur 1 und Figur 2 entsprechenden Ansicht, wenn der Spritzenkegel 42 in den Entnahmeport 10 eingeführt und die Flüssigkeitsverbindung 15 unterbrochen ist.

Der Entnahmeport 10 weist ein Gehäuse 11 auf. Der Entnahmeport 10 weist einen ersten Schlauchanschluss 12 und einen zweiten Schlauchanschluss 13 auf. Der erste Schlauchanschluss 12 kann bei Verwendung des Entnahmeports 10 zur Verbindung mit dem patientenseitigen Schenkel 3 der Schlauchleitung dienen. Der zweite Schlauchanschluss 13 kann beispielsweise zur Verbindung mit einem Reservoir dienen, das eine Mischung aus Blut und Infusionslösung enthält.

Bei dem Entnahmeport 10 münden der erste Schlauchanschluss 12 und der zweite Schlauchanschluss 13 versetzt zueinander in eine Flüssigkeitsverbindung 15 des Gehäuses. Die Flüssigkeitsverbindung 15 ermöglicht einen Flüssigkeitsstrom zwischen einer ersten Mündungsöffnung 22 des ersten Schlauchanschlusses 12 und einer zweiten Mündungsöffnung 23 des zweiten Schlauchanschlusses 13, wenn der Spritzenkegel 42 nicht in den Entnahmeport 10 eingeführt ist. Die Flüssigkeitsverbindung 15 kann im Wesentlichen durch die Innenwand des Gehäuses 11 definiert sein. Das Gehäuse 11 weist eine Öffnung 33 auf, über die der Spritzenkegel 42 in einen Aufnahmeabschnitt 17 innerhalb des Gehäuses 11 eingeführt werden kann. Die Öffnung kann durch einen Gehäuseabschnitt 14 definiert werden und beispielsweise kreisförmig sein. Wie noch ausführlicher beschrieben wird, wird die Flüssigkeitsverbindung 15 automatisch unterbrochen, wenn der Spritzenkegel 42 bis zu seinem Anschlag in den Entnahmeport 10 eingeführt wird.

Die Öffnung 33 des Gehäuses kann mit einer geeigneten Dichtung 28 versehen sein. Beispielsweise kann die Dichtung 28 an der Öffnung angebracht sein, um die Öffnung in Art eines Deckels zu verschließen. Die Dichtung 28 kann einen in den Schnittansichten der Figur 1 und der Figur 3 dargestellten Schlitz 30 aufweisen, der durch die elastischen Eigenschaften der Dichtung 28 verschlossen wird, wenn der Spritzenkegel 42 nicht in den Entnahmeport 10 eingeführt ist. Die Dichtung 28 kann durch Aufschnappen, Ultraschallschweißen oder auf andere Weise an dem Gehäuse 11 befestigt sein. Beispielsweise kann eine Halterung 27 die Dichtung 28 dichtend an ihrem Platz halten. Die Dichtung 28 kann aus einem elastomeren Material bestehen und beispielsweise eine Elastomer-Membran sein. Die Dichtung 28 kann beispielsweise Silikon, Latex, ein thermoplastisches Elastomer (TPE) und/oder Ethylen-Propylen-Dien-Kautschuk (EPDM) aufweisen. Eine einen Vorsprung bildende Außenoberfläche 29 der Dichtung 28 kann unter Vorspannung an einer Außenoberfläche des Spritzenkegels 42 anliegen, wenn der Spritzenkegel 42 in den Aufnahmeabschnitt 17 eingeführt ist. Die Dichtung 28 kann im Wesentlichen flüssigkeitsdicht an der Außenoberfläche des Spritzenkegels 42 anliegen, wenn der Spritzenkegel 42 in den Aufnahmeabschnitt 17 eingeführt ist, um einen Austritt von Flüssigkeit, insbesondere Blut, zu verhindern.

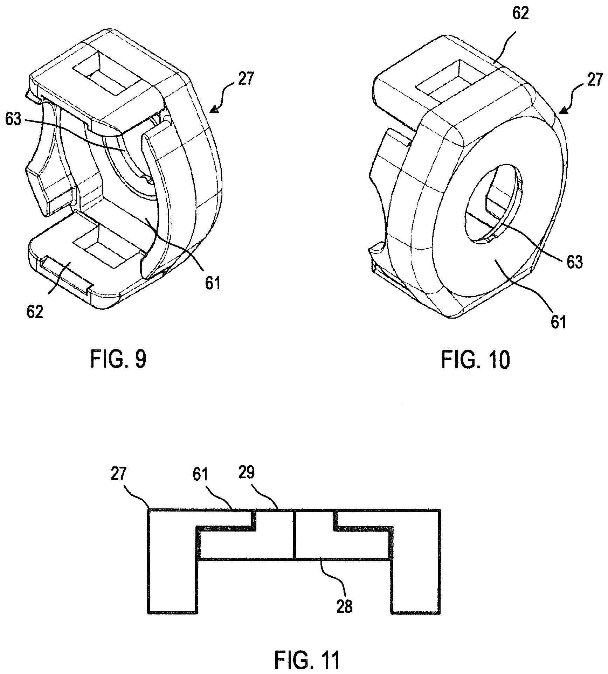

Figur 9 bis Figur 11 veranschaulichen eine mögliche Ausgestaltung der Dichtung 28 und der Halterung 27 des Entnahmeports nach einem Ausführungsbeispiel. Die Halterung 27 kann als Deckel ausgestaltet sein, der einen ringförmigen Abschnitt aufweist, der die Dichtung 28 teilweise übergreift. Die Halterung 27 kann eine Ringschulter 61 aufweisen. Die Halterung 27 kann einen Montageabschnitt 62 zur Befestigung an dem Gehäuse 11, beispielsweise durch eine Rastverbindung, aufweisen.

Die Öffnung 63 zum Einführen des Spritzenkegels 42 in das Gehäuse 11 kann von der Ringschulter 61 umgeben sein. Im montierten Zustand der Dichtung 28 und der Halterung 27 ragt ein Vorsprung 34 der Dichtung 28, beispielsweise eine Nase, in die Öffnung 63 hinein. Der Vorsprung 34 der Dichtung 28 ist so bemessen, dass er bündig mit dem die Öffnung 63 umgebenden Bereich der Halterung 27 ist, wenn der Spritzenkegel 42 nicht in den Entnahmeport 10 eingeführt ist. Der Vorsprung der Dichtung 28 ist so bemessen, dass er zusammen mit der Halterung 27 eine im Wesentlichen stufenlose Außenoberfläche um die und an der Öffnung 63 bereitstellt. Dadurch kann eine Wischdesinfektion erleichtert werden. Der Vorsprung der Dichtung 28 kann so bemessen sein, dass er die Öffnung 63 der Halterung 27 vollständig ohne Vertiefung ausfüllt und zusammen mit der Halterung 27 eine stufenlose Außenoberfläche des Entnahmeports bildet, wenn der Spritzenkegel 42 nicht in den Entnahmeport 10 eingeführt ist.

Bei weiteren Ausführungsbeispielen muss keine vom Gehäuse 11 separate Halterung vorgesehen sein. Die Dichtung 28 weist einen Vorsprung 34 auf, der in eine Öffnung des Gehäuses 11 hineinragt. Der Vorsprung 34 der Dichtung 28 ist so bemessen, dass er bündig mit dem die Öffnung umgebenden Bereich des Gehäuses 11 ist, wenn der Spritzenkegel 42 nicht in den Entnahmeport eingeführt ist. Der Vorsprung 34 der Dichtung 28 ist so bemessen, dass er zusammen mit dem Gehäuse 11 eine im Wesentlichen stufenlose Oberfläche um die Öffnung des Gehäuses 11 bereitstellt. Dadurch kann eine Wischdesinfektion erleichtert werden. Der Vorsprung 34 der Dichtung 28 kann so bemessen sein, dass er die Öffnung des Gehäuses vollständig ohne Vertiefung ausfüllt und eine stufenlose Außenoberfläche des Entnahmeports bildet, wenn der Spritzenkegel 42 nicht in den Entnahmeport eingeführt ist.

Die Halterung 27 kann eingerichtet sein, um den Spritzenkegel 42 an einem vom Endbereich 43 des Spritzenkegels beabstandeten Abschnitt des Spritzenkegels 42 klemmend zu halten. Die Halterung 27 kann insbesondere eingerichtet sein, um den Spritzenkegel 42 am proximalen Ende des Spritzenkegels 42 klemmend zu halten. Dabei kann die Halterung 27 eine nach innen gerichtete Klemmkraft auf den Spritzenkegel 42 ausüben. Die Klemmkraft kann im Wesentlichen radial zur Mittelachse des Spritzenkegels 42 gerichtet sein.

Die Halterung 27 kann sich ähnlich wie eine Klemmscheibe auf einer Welle verhalten. Die Halterung 27 kann im Gebrauch leicht elastisch einfedern und zu einer Verklemmung beim Zurückweichen des Spritzenkegels führen.

Alternativ oder zusätzlich kann der Entnahmeport 10 so eingerichtet sein, dass die Dichtung 28 die Spritze an einem vom Endbereich 43 des Spritzenkegels beabstandeten Abschnitt des Spritzenkegels 42 klemmend hält. Dabei kann die Dichtung 28 eine nach innen gerichtete Klemmkraft auf den Spritzenkegel 42 ausüben. Die Klemmkraft kann im Wesentlichen radial zur Mittelachse des Spritzenkegels 42 gerichtet sein.

Der Entnahmeport 10 ist somit eingerichtet, um den Spritzenkegel 42 klemmend an dem Entnahmeport 10 zu halten, wenn der Spritzenkegel 42 in den Entnahmeport 10 eingeführt ist. Der Entnahmeport 10 ist so ausgestaltet, dass der Spritzenkegel 42 vom Entnahmeport 10 gehalten wird, ohne mit dem Entnahmeport verschraubt werden zu müssen. Der Entnahmeport 10 kann eine von Gewindegängen freie Außenoberfläche aufweisen. Die Desinfektion der Außenoberflächen in der Nähe der Öffnung, über die der Spritzenkegel 42 eingeführt wird, wird dadurch erleichtert. Der Entnahmeport 10 kann eingerichtet sein, die Spritze mit klemmender Wirkung am Entnahmeport 10 zu halten, ohne dass die Spritze durch den Benutzer nach dem Einführen weiter aktiv in Richtung des Entnahmeports 10 gedrückt werden muss, um ein unbeabsichtigtes Lösen der Spritze vom Entnahmeport 10 zu verhindern.

Der Entnahmeport 10 weist einen Verschlussabschnitt auf, der im Gebrauch des Entnahmeports 10 mit dem Spritzenkegel 42 zusammenwirkt, um die Flüssigkeitsverbindung 15 zwischen den Mündungsöffnungen 22, 23 der Schlauchanschlüsse 12, 13 automatisch zu unterbrechen und gleichzeitig eine fluidische Kommunikation, insbesondere eine Flüssigkeitskommunikation, zwischen dem Spritzenkegel 42 und dem ersten Schlauchanschluss 12 sicherzustellen, wenn der Spritzenkegel 42 in den Entnahmeport 10 eingeführt wird. Der Verschlussabschnitt wird durch eine Dichtfläche 19 gebildet, die an einem sich verjüngenden Gehäuseabschnitt vorgesehen ist. Wenn der Spritzenkegel 42 wie in Figur 3 und Figur 4 dargestellt mit der Dichtfläche 19 in Kontakt tritt, wird die Flüssigkeitsverbindung 15 unterbrochen. Gleichzeitig kann der Spritzenkegel 42 über einen Teil 18 der Flüssigkeitsverbindung 15 mit dem ersten Schlauchanschluss 12 so kommunizieren, dass Flüssigkeit zwischen dem ersten Schlauchanschluss 12 und dem Spritzenkegel fließen kann. Blut kann über den ersten Schlauchanschluss 12 aus dem ersten Schenkel 2 der Schlauchleitung abgezogen werden.

Die Öffnung des Entnahmeports 10 und/oder an der Öffnung vorgesehene Führungselemente wie Rippen oder andere Vorsprünge definieren eine Einführrichtung 26, entlang der der Spritzenkegel 42 in den Entnahmeport 10 einführbar ist. Der erste Schlauchanschluss 12 und der zweite Schlauchanschluss 13 sind so angeordnet, dass sie entlang der Einführrichtung 26 zueinander versetzt sind. Eine erste Längsachse 24 des ersten Schlauchanschlusses 12 und eine zweite Längsachse 25 des zweiten Schlauchanschlusses 13 können entlang der Einführrichtung 26 zueinander versetzt sein. Die erste Längsachse 24 des ersten Schlauchanschlusses 12 und die zweite Längsachse 25 des zweiten Schlauchanschlusses 13 können parallel zueinander verlaufen. Das Gehäuse 11 kann eine Schulter 16 ausbilden, an der sich ein Innenquerschnitt des Gehäuses verjüngt, um sicherzustellen, dass die Dichtfläche 19 satt und im Wesentlichen flüssigkeitsdicht an einem Endbereich 43 des Spritzenkegels 42 anliegt. Der Spritzenkegel 42 kann an seinem Endbereich 43 innerhalb des Gehäuses reibschlüssig gehalten, insbesondere von dem Dichtbereich 19 festgeklemmt werden. Alternativ oder zusätzlich kann der Spritzenkegel 42 von der Dichtung 28 und/oder der Öffnung im Gehäuse 11, über die der Spritzenkegel 42 eingeführt ist, festgeklemmt werden.

Bei dem Entnahmeport 10 lenkt das Gehäuse 11 aufgrund der zueinander versetzten Anordnung des ersten Schlauchanschlusses 12 und des zweiten Schlauchanschlusses 13 den Flüssigkeitsstrom zwischen dem ersten Schlauchanschlusses 12 und dem zweiten Schlauchanschluss 13 um, wenn der Spritzenkegel 42 nicht bis zu einem im Wesentlichen dichten Kontakt mit der Dichtfläche 19 in den Entnahmeport 10 eingeführt ist. Wird der Spritzenkegel 42 durch die Dichtung 28 hindurch gedrückt, so drückt sich diese teils zur Seite, teils kann sie sich in das Gehäuse 11 einstülpen, wie dies in Figur 4 dargestellt ist. Sobald der Spritzenkegel 42 bis zu seinem Anschlag eingedrückt ist, ragt die Spitze des Spritzenkegels 42 derart in die Flüssigkeitsverbindung 15, dass die Flüssigkeitsverbindung 15 unterbrochen ist. Dadurch wird als Reaktion auf das Einführen des Spritzenkegels 42 die Flüssigkeitsverbindung zwischen dem ersten und zweiten Schlauchanschluss 12, 13 automatisch unterbrochen, und eine Aspiration von Flüssigkeit in die Spritze kann nur noch über den ersten Schlauchanschluss 12 erfolgen. Ein Einziehen einer Mischung von Blut und Infusionslösung oder einer anderen Flüssigkeit über den zweiten Schlauchanschluss 13 ist nicht möglich.

Der Entnahmeport 10 ist so ausgestaltet, dass der Strömungsquerschnitt von dem ersten Schlauchanschluss 12 und der Öffnung der Spritze nicht eingeschränkt oder reduziert wird, wenn der Spritzenkegel 42 in den Entnahmeport 10 eingeführt ist. Wie in Figur 3 und Figur 4 dargestellt, wird beim Einführen des Spritzenkegels 42 der Strömungspfad zwischen dem ersten Schlauchanschluss und dem Aufnahmeabschnitt 17 nicht verringert. Darüber hinaus überdeckt weder die Dichtung 28 noch ein anderes Element des Entnahmeports 10 die Öffnung am Ende des Spritzenkegels 42, wenn der Spritzenkegel 42 vollständig eingeführt ist. Insbesondere kann die Dichtung 28 so ausgestaltet sein, dass sie nur an der konischen Außenoberfläche des Spritzenkegels 42 anliegt, dabei aber von der axialen Endfläche des Spritzenkegels 42 beabstandet bleibt und insbesondere die Öffnung am Ende des Spritzenkegels 42 nicht einmal teilweise überdeckt, wenn der Spritzenkegel 42 vollständig eingeführt ist.

Wird der Spritzenkegel 42 wieder herausgezogen, wird die Flüssigkeitsverbindung 15 automatisch wieder geöffnet. Der Anwender muss folglich keine zusätzlichen Schritte unternehmen, um die Flüssigkeitsverbindung 15 durch eine separate manuelle Betätigung eines Dreiwegehahns zu schließen und zu öffnen.

Der Entnahmeport 10 kann so ausgestaltet sein, dass sich nach Blutabnahme aus der Schlauchleitung der Flüssigkeitspfad zwischen dem ersten Schlauchanschluss 12 und dem zweiten Schlauchanschluss 13 von selbst wieder freispült. Dazu kann eine Nase im patientenfernen Eingang vorgesehen sein. Der Entnahmeport kann so ausgestaltet sein, dass im Inneren des Entnahmeports 10 keine Volumina vorhanden sind, an denen die Strömungsgeschwindigkeit Null ist, wenn Flüssigkeit zwischen dem ersten Schlauchanschluss 12 und dem zweiten Schlauchanschluss 13 fließt.

Der Entnahmeport 10 kann zur besonders einfachen Handhabung eine Halteplatte 20 aufweisen. Die Halteplatte 20 erlaubt es, den Entnahmeport 10 beispielsweise zwischen zwei Fingern festzuhalten, während die Spritze 41 zur Blutabnahme eingeführt wird.

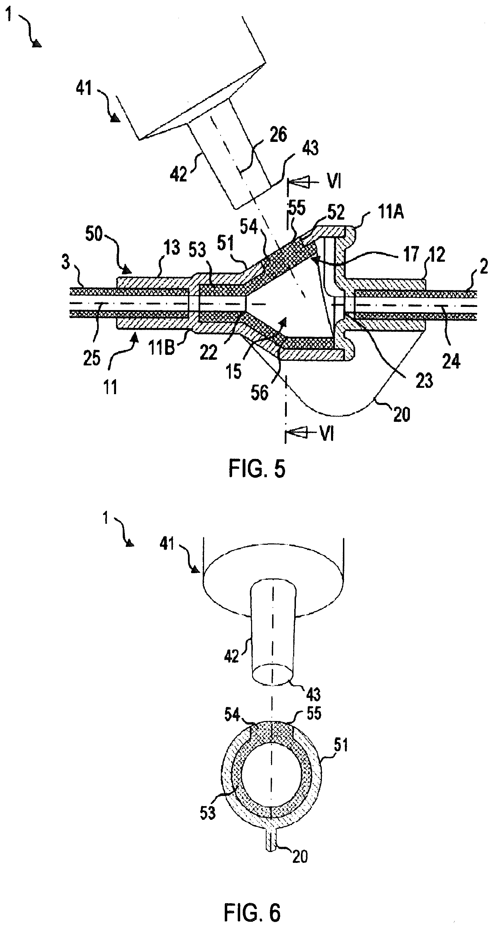

Die Figuren 5 bis 8 zeigen ein System 1 mit einem Entnahmeport 50 nach einer weiteren Ausführungsform. Elemente, die in ihrer Ausgestaltung oder Funktion Elementen entsprechen, die bereits unter Bezugnahme auf die Figuren 1 bis 4 beschrieben wurde, sind mit denselben Bezugszeichen bezeichnet.

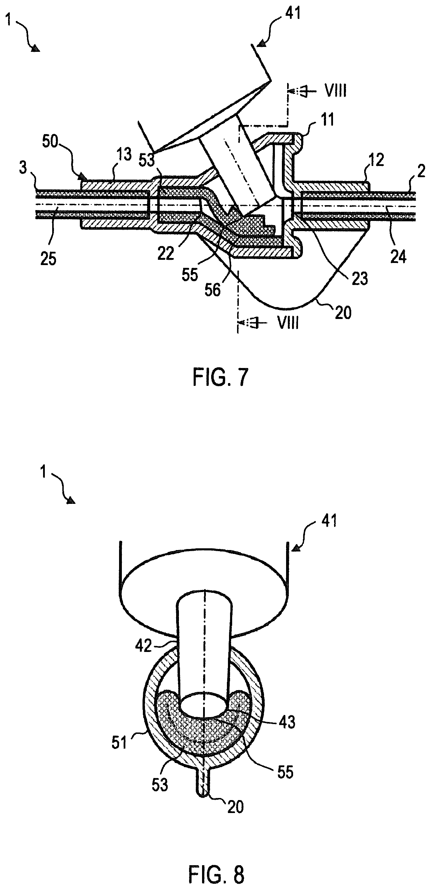

Figur 5 zeigt den Entnahmeport 50 in einer Schnittansicht, wobei die Schnittebene parallel zu Längsachsen 24, 25 von Schlauchanschlüssen 12, 13 verläuft und das System 1 in einem Zustand dargestellt ist, in dem eine Flüssigkeitsverbindung 15 offen ist. Figur 6 zeigt den Entnahmeport 50 in einer Schnittansicht, wobei die Schnittebene senkrecht zu den Längsachsen 24, 25 der Schlauchanschlüsse 12, 13 verläuft und das System 1 in einem Zustand dargestellt ist, in dem die Flüssigkeitsverbindung 15 offen ist. Figur 7 und Figur 8 zeigen den Entnahmeport 50 in einer den Schnittansichten von Figur 5 und Figur 6 entsprechenden Ansicht, wenn die Flüssigkeitsverbindung 15 geschlossen ist. Wie noch ausführlicher beschrieben wird, weist der Entnahmeport 50 einen Verschlussabschnitt 53 auf, der bei einem Einführen des Spritzenkegels 42 in den Entnahmeport 50 so verformt wird, dass der Verschlussabschnitt 53 den zweiten Schlauchanschluss 13 verschließt.

Der Entnahmeport 50 weist ein Gehäuse 11 auf. Das Gehäuse 11 kann mehrteilig sein. Beispielsweise kann das Gehäuse 11 zwei Gehäuseteile 11A, 11B umfassen, wobei an jedem der Gehäuseteile 11A, 11B jeweils einer der Schlauchanschlüsse 12, 13 angeordnet sein kann. Die ersten und zweiten Schlauchanschlüsse 12, 13 können beispielsweise koaxial angeordnet sein, so dass die erste Längsachse 24 des ersten Schlauchanschlusses 2 und die zweite Längsachse 25 des zweiten Schlauchanschlusses 3 sich entlang derselben Gerade erstrecken.

Das Gehäuse 11 weist eine Öffnung 52 auf, über die der Spritzenkegel 42 einführbar ist. Die Öffnung 52 kann, optional in Kombination mit Rippen oder anderen Führungselementen an der Öffnung 52, eine Einführrichtung 26 des Spritzenkegels 42 festlegen. Das Gehäuse 11 kann einen sich in Richtung zum zweiten Schlauchanschluss 13 hin verjüngenden Wandungsabschnitt 51 aufweisen. Jedenfalls in dem sich verjüngenden Wandungsabschnitt 51 kann ein Verschlussabschnitt 53 innerhalb des Gehäuses 11 angeordnet sein. Der Verschlussabschnitt 53 kann ein Elastomer, beispielsweise Silikon, Latex, TPE und/oder EPDM, aufweisen oder daraus bestehen. Der Verschlussabschnitt 53 kann vorteilhaft einen Vorsprung 54 aufweisen, der in der Öffnung 52 aufnehmbar ist, um die Öffnung 52 zu verschließen und/oder abzudichten, wenn der Spritzenkegel 42 nicht in den Entnahmeport 50 eingeführt ist. Eine Außenoberfläche des Vorsprungs 54, der in Art einer Nase in die Öffnung 52 hineinragt, kann dabei im Wesentlichen bündig mit einer Außenoberfläche des Gehäuses 11 sein.

Der Vorsprung 54 kann so bemessen sein, dass er bündig mit dem die Öffnung umgebenden Bereich des Gehäuses 11 ist, wenn der Spritzenkegel 42 nicht in den Entnahmeport eingeführt ist. Der Vorsprung 54 kann so bemessen sein, dass er zusammen mit dem Gehäuse 11 eine im Wesentlichen stufenlose Oberfläche um die Öffnung des Gehäuses 11 bereitstellt. Dadurch kann eine Wischdesinfektion erleichtert werden. Der Vorsprung 54 kann so bemessen sein, dass er die Öffnung des Gehäuses 11 vollständig ausfüllt und eine stufenlose Außenoberfläche des Entnahmeports 50 bildet, wenn der Spritzenkegel 42 nicht in den Entnahmeport 50 eingeführt ist.

Das Gehäuse 11 oder eine optional am Gehäuse 11 vorgesehene Halterung kann eingerichtet sein, um den Spritzenkegel 42 an einem vom Endbereich 43 des Spritzenkegels beabstandeten Abschnitt des Spritzenkegels 42 klemmend zu halten. Das Gehäuse 11 oder die Halterung kann insbesondere eingerichtet sein, um den Spritzenkegel 42 am proximalen Ende des Spritzenkegels 42 klemmend zu halten. Dabei kann eine nach innen gerichtete Klemmkraft auf den Spritzenkegel 42 ausgeübt werden. Die Klemmkraft kann im Wesentlichen radial zur Mittelachse des Spritzenkegels 42 gerichtet sein.

Der Entnahmeport 50 kann somit eingerichtet sein, den Spritzenkegel 42 klemmend an dem Entnahmeport 50 zu halten, wenn der Spritzenkegel 42 in den Entnahmeport 50 eingeführt ist. Der Entnahmeport 50 kann so ausgestaltet sein, dass der Spritzenkegel 42 vom Entnahmeport 50 klemmend gehalten wird, ohne mit dem Entnahmeport verschraubt werden zu müssen. Der Entnahmeport 50 kann eine von Gewindegängen freie Außenoberfläche aufweisen. Die Desinfektion der Außenoberflächen in der Nähe der Öffnung, über die der Spritzenkegel 42 eingeführt wird, wird dadurch erleichtert. Der Entnahmeport 50 kann eingerichtet sein, die Spritze mit klemmender Wirkung am Entnahmeport 50 zu halten, ohne dass die Spritze durch den Benutzer nach dem Einführen weiter aktiv in Richtung des Entnahmeports 50 gedrückt werden muss, um ein unbeabsichtigtes Lösen der Spritze vom Entnahmeport 50 zu verhindern.

Wie am besten in der Schnittansicht der Figur 6 ersichtlich ist, kann der Verschlussabschnitt 53 die Längsachse 25 des zweiten Schlauchanschlusses 13 ringförmig umgeben, wenn der Spritzenkegel 42 nicht in den Entnahmeport 50 eingeführt ist. Das Einführen des Spritzenkegels 42 bis zu seinem Anschlag deformiert den Verschlussabschnitt 53 elastisch und reversibel so, dass der von dem Spritzenkegel 42 verformte Verschlussabschnitt 53 den zweiten Schlauchanschluss 13 verschließt. Eine Flüssigkeitskommunikation zwischen dem Spritzenkegel 42 und dem ersten Schlauchanschluss 12 bleibt möglich.

Die Einführrichtung 26, entlang der der Spritzenkegel 42 in den Entnahmeport 50 einführbar ist, kann relativ zu den Längsachsen des ersten Schlauchanschlusses 12 und des zweiten Schlauchanschlusses 13 geneigt sein. Dabei kann die Einführrichtung 26 einen stumpfen Winkel mit der Längsachse 24 des ersten Schlauchanschlusses 12 einschließen, um eine gute Flüssigkeitskommunikation zwischen dem Spritzenkegel 42 und dem ersten Schlauchanschluss 12 sicherzustellen, selbst wenn der Verschlussabschnitt 53 von dem Spritzenkegel 42 elastisch verformt wird. Die Einführrichtung 26 kann einen spitzen Winkel, beispielsweise von wenigstens 60°, mit der Längsachse 25 des zweiten Schlauchanschlusses 12 einschließen, um eine gute Flüssigkeitskommunikation zwischen dem Spritzenkegel 42 und dem ersten Schlauchanschluss 12 sicherzustellen, selbst wenn der Verschlussabschnitt 53 von dem Spritzenkegel 42 elastisch verformt wird.

Der Verschlussabschnitt 53 kann ausgestaltet sein, um den zweiten Schlauchanschluss 13 zu verschließen, wenn der Spritzenkegel 42 eingeführt ist, und/oder die Öffnung 52 des Gehäuses 11 zu verschließen, wenn der Spritzenkegel 42 nicht eingeführt ist. Optional kann der Verschlussabschnitt 53 allein oder zusammenwirkend mit dem Gehäuse 11 auch den Spritzenkegel 42 innerhalb des Gehäuses 11 klemmend festhalten. Beispielsweise kann ein konkaver Bereich 56 des Verschlussabschnitts 53 so positioniert sein, dass der von dem Spritzenkegel deformierte Verschlussabschnitt 53 eine Kante des Spritzenkegels 42 wenigstens teilweise umgreift und klemmend festhält.

Der Entnahmeport 50 ist so ausgestaltet, dass der Strömungsquerschnitt von dem ersten Schlauchanschluss 12 und der Öffnung der Spritze nicht wesentlich eingeschränkt oder reduziert wird, wenn der Spritzenkegel 42 in den Entnahmeport 50 eingeführt ist. Wie in Figur 7 dargestellt ist, wird beim Einführen des Spritzenkegels 42 der Strömungspfad zwischen dem ersten Schlauchanschluss und dem Aufnahmeabschnitt 17 nicht verringert. Der Verschlussabschnitt 53 kann so ausgestaltet sein, dass er nur an der konischen Außenoberfläche des Spritzenkegels 42 anliegt, die Öffnung am Ende des Spritzenkegels 42 aber allenfalls geringfügig oder vorteilhaft gar nicht überdeckt.

Wird der Spritzenkegel 42 aus dem Entnahmeport 50 herausgezogen, kehrt der Verschlussabschnitt 53 aufgrund seiner Elastizität in die in Figur 5 und Figur 6 dargestellte Konfiguration zurück. Die Flüssigkeitsverbindung 15 zwischen dem ersten Schlauchanschluss 12 und dem zweiten Schlauchanschluss 13 wird automatisch wieder geöffnet.

Der Entnahmeport 50 kann so ausgestaltet sein, dass sich nach der Blutabnahme aus der Schlauchleitung der Flüssigkeitspfad zwischen dem ersten Schlauchanschluss 12 und dem zweiten Schlauchanschluss 13 von selbst wieder freispült. Der Entnahmeport kann so ausgestaltet sein, dass im Inneren des Entnahmeports 50 keine Volumina vorhanden sind, an denen die Strömungsgeschwindigkeit Null ist, wenn Flüssigkeit zwischen dem ersten Schlauchanschluss 12 und dem zweiten Schlauchanschluss 13 fließt.

Bei den Entnahmeports nach allen Ausführungsformen ist es nicht zwingend erforderlich, dass der zweite Schlauchanschluss vollständig flüssigkeitsdicht verschlossen wird. Eine zuverlässige Aspiration von Blut über den ersten Schlauchanschluss wird bereits erreicht, wenn ein Strömungswiderstand für Flüssigkeit zwischen dem ersten und dem zweiten Schlauchanschluss deutlich größer als ein Strömungswiderstand für Flüssigkeit zwischen dem Spritzenkegel und dem ersten Schlauchanschluss und der Gefäßkanüle ist, wenn der Spritzenkegel bis zu seinem Anschlag in den Entnahmeport eingeführt ist.

Entnahmeports nach allen Ausführungsbeispielen können beispielsweise so ausgestaltet sein, dass der erste Schlauchanschluss und der zweite Schlauchanschluss jeweils mit Schenkeln einer Schlauchleitung verbindbar sind, wie sie in medizinischen Anwendungen üblich sind. Der erste Schlauchanschluss und der zweite Schlauchanschluss können beispielsweise jeweils ausgestaltet sein, um mit Leitungen mit einem Außendurchmesser von 2 mm bis einschließlich 4,5 mm, aber auch mit Leitungen anderer Abmessungen verbunden zu werden.

Der Entnahmeport nach verschiedenen Ausführungsbeispielen ermöglicht, dass die Spritze klemmend am Entnahmeport festgehalten wird, ohne dass eine Luer-Lock-Verschraubung verwendet werden muss. Dies erlaubt die Verwendung des Entnahmeports mit einer Vielzahl von Spritzen, insbesondere auch mit Spritzen, die keine Luer-Lock-Verbinder aufweisen.

Der Entnahmeport nach verschiedenen Ausführungsbeispielen bildet eine stufenlose Außenoberfläche an der Öffnung, über die der Spritzenkegel in den Entnahmeport eingeführt wird. Im Vergleich zu einem Entnahmeport, der dauerhaft eine Vertiefung an der Öffnung aufweist, wird dadurch die Desinfektion des Entnahmeports vereinfacht. Insbesondere kann in einfacher Weise eine Wischdesinfektion ausgeführt werden.

Der Entnahmeport nach verschiedenen Ausführungsbeispielen erlaubt auch eine Aspiration von Blut in die Spritze ohne Einengung des Strömungsquerschnitts. Dadurch wird eine effiziente Blutentnahme ermöglicht, die das Risiko vermindert, dass durch auftretende Unterdrücke die roten Blutkörperchen geschädigt werden.

Während Ausführungsbeispiele unter Bezugnahme auf die Figuren beschrieben wurden, können bei weiteren Ausführungsbeispielen zahlreiche Abwandlungen implementiert werden. Beispielsweise können Verschlussabschnitte, die reversibel lösbar mit einem Spritzenkegel in Kontakt treten und als Reaktion darauf die Flüssigkeitsverbindung zwischen dem ersten und dem zweiten Schlauchanschluss unterbrechen, auch andere als die detailliert beschriebenen Ausgestaltungen aufweisen.

Mit dem erfindungsgemäßen Entnahmeport wird das Risiko, das mit einer fehlerhaften Bedienung herkömmlicher Dreiwegehähne verbunden ist, wesentlich reduziert.

## Patentansprüche

1. Entnahmeport (10; 50) zum Entnehmen einer Flüssigkeit, insbesondere Blut, aus einer Schlauchleitung, aufweisend:
ein Gehäuse (11);
einen ersten Schlauchanschluss (12), der mit einem Schenkel (2) einer Schlauchleitung verbunden oder verbindbar ist;
einen zweiten Schlauchanschluss (13);
eine Fluidverbindung (15), insbesondere eine Flüssigkeitsverbindung, zwischen dem ersten und zweiten Schlauchanschluss (12, 13); und
einen Aufnahmeabschnitt (17), der dazu geeignet ist, einen Spritzenabschnitt, insbesondere einen Spritzenkegel (42), einer Spritze (41) reversibel lösbar aufzunehmen,
wobei der Entnahmeport (10; 50) derart ausgestaltet ist, dass ein Einführen des Spritzenabschnitts in den Aufnahmeabschnitt (17) die Fluidverbindung (15) zwischen dem ersten Schlauchanschluss (12) und dem zweiten Schlauchanschluss (13) unterbricht und eine fluidische Kommunikation des Spritzenabschnitts mit dem ersten Schlauchanschluss (12) bewirkt, und wobei der Entnahmeport (10; 50) eingerichtet ist, den Spritzenabschnitt mit einer radial nach innen gerichteten klemmenden Kraft am Entnahmeport (10; 50) zu halten, wenn der Spritzenabschnitt in den Aufnahmeabschnitt (17) eingeführt ist,
wobei das Gehäuse (11) eine Öffnung (33; 52) aufweist, über die der Spritzenabschnitt in den Aufnahmeabschnitt (17) des Entnahmeports (10; 50) einführbar ist, und
wobei der Entnahmeport (10; 50) eine an der Öffnung (33; 52) angeordnete Dichtung (28) aufweist,
wobei die Dichtung (28) einen Vorsprung (34; 54) aufweist, der in die Öffnung (33; 52) so hineinragt, dass eine Außenoberfläche der Dichtung (28) weitgehend bündig mit dem Gehäuse oder mit einer Halterung (27) der Dichtung (28) ist, wenn der Spritzenabschnitt nicht in das Gehäuse eingefügt ist,
wobei die mit dem Gehäuse (11) oder der Halterung (27) weitgehend bündige Außenoberfläche (29; 55) der Dichtung (28) eine stufenarme Außenoberfläche des Entnahmeports (10; 50) für eine Wischdesinfektion bildet.

2. Entnahmeport (10; 50) nach Anspruch 1, wobei der Vorsprung (34; 54) der Dichtung (28) so bemessen ist, dass er die Öffnung (33; 52) des Gehäuses (11) vollständig ohne Vertiefung ausfüllt, wenn der Spritzenabschnitt nicht in den Entnahmeport (10; 50) eingeführt ist

3. Entnahmeport (10; 50) nach Anspruch 1 oder Anspruch 2, ferner mit:
einem Verschlussabschnitt (19; 53), der an dem Gehäuse (11) ausgebildet oder angebracht ist, wobei der Verschlussabschnitt (19; 53) eingerichtet ist, um mit dem Spritzenabschnitt reversibel lösbar in Kontakt zu treten, um die Fluidverbindung (15) zwischen dem ersten Schlauchanschluss (12) und dem zweiten Schlauchanschluss (13) zu unterbrechen.

4. Entnahmeport (10; 50) nach Anspruch 3,
wobei die Dichtung (28) und/oder das Gehäuse (11) an der Öffnung (33; 52) für einen dichtenden Eingriff mit Spritzenkegeln (42) unterschiedlicher Durchmesser elastisch verformbar ist,
wobei optional die Dichtung eine Nase (34; 54) für eine abdichtende Anlage an einer Oberfläche des Gehäuses (11) aufweist, und/oder
wobei optional das Gehäuse (11) und/oder die Dichtung (28) eingerichtet ist, um den Spritzenabschnitt an einem von einem Ende (43) des Spritzenkegels (42) beabstandeten Abschnitt des Spritzenkegels (42) reibschlüssig, insbesondere mit klemmender Wirkung, zu halten.

5. Entnahmeport (10; 50) nach Anspruch 3 oder 4, ferner mit
einer von dem Gehäuse (11) separaten Halterung (27) zum Halten der Dichtung (28),
wobei optional die Halterung (27) eingerichtet ist, einen in den Aufnahmeabschnitt (17) eingeführten Spritzenkegel (42) klemmend zu halten, und/oder
wobei optional die Halterung (27) eine die Dichtung (28) teilweise übergreifende Ringschulter aufweist, die eingerichtet ist, den in den Aufnahmeabschnitt (17) eingeführten Spritzenkegel (42) klemmend zu halten.

6. Entnahmeport (10; 50) nach einem der Ansprüche 3 bis 5,
wobei der Verschlussabschnitt (19) eine an dem Gehäuse (11) des Entnahmeports (10; 50) ausgebildete Dichtfläche (19) aufweist, um durch einen Kontakt des Spritzenabschnitts mit der Dichtfläche (19) den zweiten Schlauchanschluss (13) zu verschließen,
wobei optional der erste Schlauchanschluss (12) eine erste Längsachse (24) und der zweite Schlauchanschluss (13) eine zu der ersten Längsachse (24) versetzte zweite Längsachse (25) aufweist,
wobei optional das Gehäuse (11) ausgestaltet ist, um einen Flüssigkeitsstrom zwischen dem ersten Schlauchanschluss (12) und dem zweiten Schlauchanschluss (13) im Bereich des Aufnahmeabschnitts (17) und/oder im Aufnahmeabschnitt (17) umzulenken, wenn der Spritzenabschnitt nicht in den Aufnahmeabschnitt (17) eingeführt ist.

7. Entnahmeport (10; 50) nach einem der Ansprüche 3 bis 6,
wobei der Verschlussabschnitt (53) elastisch verformbar ist, um bei dem Einführen des Spritzenabschnitts in den Aufnahmeabschnitt (17) den zweiten Schlauchanschluss (13) zu verschließen,
wobei optional der elastisch verformbare Verschlussabschnitt (53) durch das Einführen des Spritzenabschnitts in den Aufnahmeabschnitt (17) reversibel aus einer ersten Konfiguration, in der der Verschlussabschnitt (53) sowohl den ersten Schlauchanschluss (12) als auch den zweiten Schlauchanschluss (13) frei lässt, in eine davon verschiedene zweite Konfiguration, in der der elastisch verformbare Verschlussabschnitt (53) den zweiten Schlauchanschluss (13) verschließt, überführbar ist,
wobei optional das Gehäuse (11) eine Öffnung (52) aufweist, über die der Spritzenabschnitt in den Aufnahmeabschnitt (17) des Entnahmeports (10; 50) einführbar ist,
wobei der elastisch verformbare Verschlussabschnitt (53) eingerichtet ist, um in der ersten Konfiguration die Öffnung (52) zu verschließen und/oder abzudichten.

8. Entnahmeport (10; 50) nach Anspruch 7,
wobei der elastisch verformbare Verschlussabschnitt (53) aus einem elastomeren Material besteht.

9. Entnahmeport (10; 50) nach einem der Ansprüche 3 bis 8,
wobei der Verschlussabschnitt (19; 53) eingerichtet ist, um den in den Aufnahmeabschnitt (17) eingeführten Spritzenabschnitt reibschlüssig, insbesondere mit klemmender Wirkung, zu halten, und/oder
wobei der Entnahmeport (10; 50) eingerichtet ist, um den Spritzenabschnitt wenigstens an einem Endbereich (43) des Spritzenabschnitts innerhalb des Gehäuses (11) reibschlüssig, insbesondere mit klemmender Wirkung, zu halten.

10. Entnahmeport (10; 50) nach einem Ansprüche 3 bis 9,
wobei das Gehäuse (11) Vorsprünge, insbesondere Rippen, zum Führen oder reibschlüssigen Halten des Spritzenabschnitts aufweist.

11. Entnahmeport (10; 50) nach einem der vorhergehenden Ansprüche, ferner mit:
wenigstens eine Halteplatte (20) zum Halten und/oder Fixieren des Entnahmeports (10; 50) auf der Haut eines Patienten oder an einer anderen Struktur.

12. Entnahmeport (10; 50) nach einem der vorhergehenden Ansprüche,
wobei ein Strömungsquerschnitt einer Fluidverbindung zwischen dem ersten Schlauchabschnitt (12) und dem Aufnahmeabschnitt (17) unverändert bleibt, wenn der Spritzenabschnitt in den Aufnahmeabschnitt (17) eingeführt wird.

13. Entnahmeport (10; 50) nach einem der vorhergehenden Ansprüche,
wobei der Entnahmeport (10; 50) ausgestaltet ist, um nach Entfernen des Spritzenabschnitts aus dem Aufnahmeabschnitt (17) automatisch freigespült zu werden.

14. System (1), aufweisend:
einen Entnahmeport (10; 50) nach einem der vorhergehenden Ansprüche; und
eine Spritze (41) mit einem Spritzenkegel (42), der dazu geeignet ist, zum Entnehmen von Blut aus einer Schlauchleitung über den Entnahmeport (10; 50) reversibel lösbar in den Aufnahmeabschnitt (17) des Entnahmeports (10; 50) eingeführt zu werden,
wobei optional das System (1) durch ein Einführen des Spritzenkegels (42) in den Aufnahmeabschnitt (17) zwischen einer ersten Konfiguration, in der der Spritzenkegel (42) außerhalb des Aufnahmeabschnitts (17) angeordnet ist und die Fluidverbindung (15) zwischen dem ersten Schlauchanschluss (12) und dem zweiten Schlauchanschluss (13) offen ist, und einer zweiten Konfiguration überführbar ist, in der der Spritzenkegel (42) in den Aufnahmeabschnitt (17) des Entnahmeports (10; 50) eingeführt ist, die Fluidverbindung (15) zwischen dem ersten Schlauchanschluss (12) und dem zweiten Schlauchanschluss (13) unterbrochen ist und der Spritzenkegel (42) mit dem ersten Schlauchanschluss (12) kommuniziert.

15. Verwendung des Entnahmeports (10; 50) nach einem der Ansprüche 1 bis 13 zum Entnehmen von Blut aus einem Schenkel (2) einer Schlauchleitung.

## Claims

1. A withdrawal port (10; 50) for withdrawing a liquid, in particular blood, from a tubing, comprising:
a housing (11);
a first tube connection (12) which is connected or connectable with an arm (2) of a tubing;
a second tube connection (13);
a fluid connection (15), in particular a liquid connection, between the first and second tube connections (12, 13); and
a receiving section (17) which is suitable for reversibly releasably receiving a syringe section, particularly a syringe cone (42), of a syringe (41),
wherein the withdrawal port (10; 50) is configured such that an insertion of the syringe section into the receiving section (17) interrupts the fluid connection (15) between the first tube connection (12) and the second tube connection (13) and causes fluid communication of the syringe section with the first tube connection (12), and wherein the withdrawal port (10; 50) is configured to retain the syringe section at the withdrawal port (10; 50) with a clamping force that is directed radially inwardly when the syringe section is inserted into the receiving section (17),
wherein the housing (11) comprises an aperture (33; 52) through which the syringe section is insertable into the receiving section (17) of the withdrawal port (10; 50), and
wherein the withdrawal port (10; 50) comprises a seal (28) arranged at the aperture (33; 52),
wherein the seal (28) comprises a protrusion (34; 54) which extends into the aperture (33; 52) such that an outer surface of the seal (28) is substantially flush with the housing or with a retainer (27) of the seal (28) when the syringe section is not inserted into the housing,
wherein the outer surface (29; 55) of the seal (28), which is substantially flush with the housing (11) or the retainer (27), forms an outer surface of the withdrawal port (10; 50) without pronounced steps for wipe disinfection.

2. The withdrawal port (10; 50) according to claim 1, wherein the protrusion (34; 54) of the seal (28) is dimensioned such that it completely fills the aperture (33; 52) of the housing (11) without a recess when the syringe section is not inserted into the withdrawal port (10; 50).

3. The withdrawal port (10; 50) according to claim 1 or 2, further comprising:
a closure section (19; 53) which is formed or arranged at the housing (11), wherein the closure section (19; 53) is configured so as to reversibly releasably engage the syringe section in order to interrupt the fluid connection (15) between the first tube connection (12) and the second tube connection (13).

4. The withdrawal port (10; 50) according to claim 3,
wherein the seal (28) and/or the housing (11) is elastically deformable at the aperture (33; 52) for a sealing engagement with syringe cones (42) of different diameters,
wherein optionally the seal comprises a nose (34; 54) for sealingly abutting a surface of the housing (11), and/or
wherein optionally the housing (11) and/or the seal (28) is configured so as to frictionally retain, particularly in a clamped manner, the syringe section at a section of the syringe cone (42) spaced apart from an end (43) of the syringe cone (42).

5. The withdrawal port (10; 50) according to claim 3 or 4, further comprising
a retainer (27) separate from the housing (11) for retaining the seal (28),
wherein optionally the retainer (27) is configured to retain in a clamped manner a syringe cone (42) inserted into the receiving section (17), and/or
wherein optionally the retainer (27) comprises an annular shoulder partially engaging over the seal (28), said annular shoulder being configured to retain in a clamped manner the syringe cone (42) inserted into the receiving section (17).

6. The withdrawal port (10; 50) according to any one of claims 3 to 5,
wherein the closure section (19) comprises a sealing surface (19) formed at the housing (11) of the withdrawal port (10; 50) for closing the second tube connection (13) by way of contact of the syringe section with the sealing surface (19),
wherein optionally the first tube connection (12) comprises a first longitudinal axis (24), and the second tube connection (13) comprises a second longitudinal axis (25) offset from the first longitudinal axis (24),
wherein optionally the housing (11) is configured to redirect a liquid flow between the first tube connection (12) and the second tube connection (13) in the region of the receiving section (17) and/or in the receiving section (17) when the syringe section is not inserted into the receiving section (17).

7. The withdrawal port (10; 50) according to any one of claims 3 to 6,
wherein the closure section (53) is elastically deformable in order to close the second tube connection (13) during insertion of the syringe section into the receiving section (17),
wherein optionally, by means of inserting the syringe section into the receiving section (17), the elastically deformable closure section (53) is reversibly convertible from a first configuration in which the closure section (53) leaves open both the first tube connection (12) and the second tube connection (13) into a second configuration different from the first configuration in which the elastically deformable closure section (53) closes the second tube connection (13),
wherein optionally the housing (11) comprises an aperture (52) through which the syringe section is insertable into the receiving section (17) of the withdrawal port (10; 50),
wherein the elastically deformable closure section (53) is configured to close and/or seal the aperture (52) in the first configuration.

8. The withdrawal port (10; 50) according to claim 7,
wherein the elastically deformable closure section (53) consists of an elastomeric material.

9. The withdrawal port (10; 50) according to any one of claims 3 to 8,
wherein the closure section (19; 53) is configured so as to frictionally, in particular in a clamped manner, retain the syringe section inserted into the receiving section (17), and/or
wherein the withdrawal port (10; 50) is configured so as to frictionally, in particular in a clamped manner, retain the syringe section at at least an end region (43) of the syringe section within the housing (11).

10. The withdrawal port (10; 50) according to any one of claims 3 to 9,
wherein the housing (11) comprises protrusions, particularly ridges, for guiding or frictionally retaining the syringe section.

11. The withdrawal port (10; 50) according to any one of the preceding claims, further comprising:
at least one retaining plate (20) for retaining and/or fastening the withdrawal port (10; 50) on a patient's skin or on another structure.

12. The withdrawal port (10; 50) according to any one of the preceding claims,
wherein a flow cross-section of a fluid connection between the first tube connection (12) and the receiving section (17) remains unchanged when the syringe section is being inserted into the receiving section (17).

13. The withdrawal port (10; 50) according to any one of the preceding claims,
wherein the withdrawal port (10; 50) is configured so as to be automatically flushed after the syringe section has been removed from the receiving section (17).

14. A system (1), comprising:
a withdrawal port (10; 50) according to any one of the preceding claims; and
a syringe (41) with a syringe cone (42) suitable for being reversibly releasably inserted into the receiving section (17) of the withdrawal port (10; 50) in order to withdraw blood from a tubing by means of the withdrawal port (10; 50),
wherein optionally, by means of inserting the syringe cone (42) into the receiving section (17), the system (1) is convertible between a first configuration in which the syringe cone (42) is arranged outside of the receiving section (17) and the fluid connection (15) between the first tube connection (12) and the second tube connection (13) is open and a second configuration in which the syringe cone (42) is inserted into the receiving section (17) of the withdrawal port (10; 50), the fluid connection (15) between the first tube connection (12) and the second tube connection (13) is interrupted, and the syringe cone (42) is in communication with the first tube connection (12).

15. A use of the withdrawal port (10; 50) according to any one of claims 1 to 13 for withdrawing blood from an arm (2) of a tubing.

## Revendications

1. Orifice de prélèvement (10 ; 50) pour prélever un liquide, en particulier du sang, à partir d'un tuyau flexible, comprenant :
un boîtier (11) ;
un premier raccord de tuyau (12) qui est connecté ou peut être connecté à une branche (2) d'un tuyau flexible ;
un deuxième raccord de tuyau (13) ;
une liaison fluidique (15), en particulier une liaison liquide, entre le premier et le deuxième raccord de tuyau (12, 13) ; et
une section de réception (17) qui est adaptée pour recevoir de manière réversible et détachable une section de seringue, en particulier un cône de seringue (42), d'une seringue (41),
où l'orifice de prélèvement (10 ; 50) est configuré de telle sorte que l'insertion de la section de seringue dans la section de réception (17) interrompt la liaison fluidique (15) entre le premier raccord de tuyau (12) et le second raccord de tuyau (13) et provoque une communication fluidique de la section de seringue avec le premier raccord de tuyau (12), et où l'orifice de prélèvement (10 ; 50) est adapté pour maintenir la section de seringue contre l'orifice de prélèvement (10 ; 50) avec une force de serrage dirigée radialement vers l'intérieur lorsque la section de seringue est insérée dans la section de réception (17),
où le boîtier (11) présente une ouverture (33 ; 52) par laquelle la section de seringue peut être insérée dans la section de réception (17) de l'orifice de prélèvement (10 ; 50), et
où l'orifice de prélèvement (10 ; 50) comporte un joint (28) disposé au niveau de l'ouverture (33 ; 52),
où le joint (28) présente une saillie (34 ; 54) faisant saillie dans l'ouverture (33 ; 52) de telle sorte qu'une surface extérieure du joint (28) est sensiblement alignée avec le boîtier ou avec un support (27) du joint (28) lorsque la section de seringue n'est pas insérée dans le boîtier,
où la surface extérieure (29 ; 55) du joint (28), qui est sensiblement alignée avec le boîtier (11) ou le support (27), forme une surface extérieure de l'orifice de prélèvement (10 ; 50) ayant peu de gradins, pour une désinfection par essuyage.

2. Orifice de prélèvement (10 ; 50) selon la revendication 1, où la saillie (34 ; 54) du joint (28) est dimensionnée de manière à remplir complètement l'ouverture (33 ; 52) du boîtier (11) sans évidement lorsque la section de seringue n'est pas insérée dans l'orifice de prélèvement (10 ; 50).

3. Orifice de prélèvement (10 ; 50) selon la revendication 1 ou la revendication 2, comprenant en outre :
une section de fermeture (19 ; 53) qui est formée ou fixée au boîtier (11), où la section de fermeture (19 ; 53) est adaptée pour entrer en contact de manière réversible et détachable avec la section de seringue pour interrompre la liaison fluidique (15) entre le premier raccord de tuyau (12) et le second raccord de tuyau (13).

4. Orifice de prélèvement (10 ; 50) selon la revendication 3,
où le joint (28) et/ou le boîtier (11) sont élastiquement déformables au niveau de l'ouverture (33 ; 52) pour un engagement étanche avec des cônes de seringue (42) de différents diamètres,
où, optionnellement, le joint présente un nez (34 ; 54) pour un appui étanche sur une surface du boîtier (11), et/ou
où, optionnellement, le boîtier (11) et/ou le joint (28) est agencé pour maintenir la section de seringue, au niveau d'une section du cône de seringue (42) espacée d'une extrémité (43) du cône de seringue (42), par friction, en particulier par un effet de serrage.

5. Orifice de prélèvement (10 ; 50) selon la revendication 3 ou 4, comprenant en outre
un support (27) séparé du boîtier (11) pour maintenir le joint (28),
où optionnellement le support (27) est adapté pour serrer un cône de seringue (42) inséré dans la section de réception (17), et/ou
où optionnellement le support (27) comprend un épaulement annulaire recouvrant partiellement le joint (28), qui est adapté pour serrer le cône de seringue (42) inséré dans la section de réception (17).

6. Orifice de prélèvement (10, 50) selon l'une des revendications 3 à 5,
où la section de fermeture (19) comporte une surface d'étanchéité (19) formée sur le boîtier (11) de l'orifice de prélèvement (10 ; 50) pour fermer le deuxième raccord de tuyau (13) par contact de la section de seringue avec la surface d'étanchéité (19),
où, optionnellement, le premier raccord de tuyau (12) présente un premier axe longitudinal (24) et le deuxième raccord de tuyau (13) présente un deuxième axe longitudinal (25) décalé par rapport au premier axe longitudinal (24),
où, optionnellement, le boîtier (11) est configuré pour dévier un écoulement de liquide entre le premier raccord de tuyau (12) et le second raccord de tuyau (13) dans la zone de la section de réception (17) et/ou dans la section de réception (17) lorsque la section de seringue n'est pas insérée dans la section de réception (17).

7. Orifice de prélèvement (10 ; 50) selon l'une des revendications 3 à 6,
où la section de fermeture (53) est élastiquement déformable pour fermer le deuxième raccord de tuyau (13) lorsque la section de seringue est insérée dans la section de réception (17),
où optionnellement, en insérant la section de seringue dans la section de réception (17), la section de fermeture (53) élastiquement déformable peut être transférée de manière réversible d'une première configuration, dans laquelle la section de fermeture (53) laisse libre aussi bien le premier raccord de tuyau (12) que le second raccord de tuyau (13), à une deuxième configuration différente de la première, dans laquelle la section de fermeture (53) élastiquement déformable ferme le second raccord de tuyau (13),
où optionnellement, le boîtier (11) comporte une ouverture (52) par laquelle la section de seringue peut être insérée dans la section de réception (17) de l'ouverture de prélèvement (10 ; 50),
où la section de fermeture élastiquement déformable (53) est agencée pour fermer et/ou sceller l'ouverture (52) dans la première configuration.

8. Orifice de prélèvement (10 ; 50) selon la revendication 7,
où la section de fermeture élastiquement déformable (53) consiste en un matériau élastomère.

9. Orifice de prélèvement (10 ; 50) selon l'une des revendications 3 à 8,
où la section de fermeture (19 ; 53) est agencée pour maintenir la section de seringue insérée dans la section de réception (17) par friction, en particulier par un effet de serrage, et/ou
où l'orifice de prélèvement (10 ; 50) est agencé pour maintenir la section de seringue par friction, en particulier avec un effet de serrage, au moins à une région d'extrémité (43) de la section de seringue à l'intérieur du boîtier (11).

10. Orifice de prélèvement (10 ; 50) selon l'une des revendications 3 à 9,
où le boîtier (11) présente des saillies, en particulier des nervures, pour guider ou maintenir par friction la section de seringue.

11. Orifice de prélèvement (10 ; 50) selon l'une des revendications précédentes, comprenant en outre :
au moins une plaque de maintien (20) pour maintenir et/ou fixer l'orifice de prélèvement (10 ; 50) à la peau d'un patient ou à une autre structure.

12. Orifice de prélèvement (10 ; 50) selon l'une des revendications précédentes,
où une section transversale d'écoulement d'une liaison fluidique entre le premier raccord de tuyau (12) et la section de réception (17) reste inchangée lorsque la section de seringue est insérée dans la section de réception (17).

13. Orifice de prélèvement (10 ; 50) selon l'une des revendications précédentes,
où l'orifice de prélèvement (10 ; 50) est adapté pour être automatiquement rincé après le retrait de la section de seringue de la section de réception (17).

14. Système (1) comportant :
un orifice de prélèvement (10 ; 50) selon l'une des revendications précédentes ; et
une seringue (41) avec un cône de seringue (42) qui est adapté pour être inséré de manière réversible et détachable dans la section de réception (17) de l'orifice de prélèvement (10 ; 50) pour prélever du sang dans un tuyau flexible par l'intermédiaire de l'orifice de prélèvement (10 ; 50),
où optionnellement, par une insertion du cône de seringue (42) dans la section de réception (17), le système (1) peut être transféré d'une première configuration, dans laquelle le cône de seringue (42) est disposé en-dehors de la section de réception (17) et la liaison fluidique (15) entre le premier raccord de tuyau (12) et le second raccord de tuyau (13) est ouverte, et une deuxième configuration, dans laquelle le cône de seringue (42) est inséré dans la section de réception (17) du port de prélèvement (10 ; 50), la liaison fluidique (15) entre le premier raccord de tuyau (12) et le second raccord de tuyau (13) est interrompue et le cône de seringue (42) communique avec le premier raccord de tuyau (12).

15. Utilisation de l'orifice de prélèvement (10 ; 50) selon l'une des revendications 1 à 13 pour le prélèvement de sang dans une branche (2) d'un tuyau flexible.
